# EUROPÄISCHE PATENTANMELDUNG

(11) **EP 3 598 901 A1**
(43) Veröffentlichungstag der Anmeldung: **29.01.2020**
(21) Anmeldenummer: 18185038.9
(22) Anmeldetag: 23.07.2018
(51) Int. Cl.: A23C 9/13, A23C 9/12, A23C 9/20, A23L 33/125, A23L 33/00, A23L 29/00, A23K 20/163, C12N 9/38

(54) **BETA-GALACTOSIDASE AUS L. BULGARICUS ZUR SYNTHESE VON GALACTOOLIGOSACCHARIDEN IN MOLKE**

(71) Anmelder: optiferm GmbH, 87466 Oy-Mittelberg (DE); Hochschule Anhalt, 06366 Köthen (DE)
(72) Erfinder: Kleinschmidt, Thomas, 06386 Osternienburger Land, Großpaschleben (DE); Fischer, Christin, 06366 Köthen (DE); Weichhard, Edgar, 87466 Oy-Mittelberg (DE); Fleischner, Wilhelm, 91732 Merkendorf (DE)
(74) Vertreter: Moré, Solveig Helga

(57) **Zusammenfassung**

Die vorliegende Erfindung betrifft ein Verfahren zur Herstellung von Galactooligosacchariden (GOS) oder einer Galactooligosaccharid-haltigen Zusammensetzung, bei dem man eine von *L*. *bulgaricus* (*L*. *delbrueckii spp*. *bulgaricus*) abgeleitete beta-Galactosidase (auch Lactase [EC3.2.1.23]) bei einer Temperatur von 37°C oder weniger mit einer Lactose-haltigen Zusammensetzung wie Milch, Puffer oder Molke, z.B. Süßmolke, Sauermolke, Molkenkonzentrat oder Molkenpermeat, inkubiert, bzw. eine Verwendung des Enzyms zur Herstellung von Galactooligosacchariden oder einer Galactooligosaccharid-haltigen Zusammensetzung in einer solchen Lactose-haltigen Zusammensetzung. Ferner wird ein Verfahren zur Herstellung einer Galactooligosaccharid-haltigen Zusammensetzung mit einem definierten Anteil von DP4 Galactooligosacchariden und DP3 Galactooligosacchariden an allen Galactooligosacchariden bereitgestellt. Die Erfindung stellt ferner mit den erfindungsgemäßen Verfahren herstellbare Zusammensetzungen und dabei vorteilhaft einzusetzende, in Caseinpepton-Medium hergestellte beta-Galactosidase und Verfahren zu ihrer Herstellung zur Verfügung.

## Beschreibung

Die vorliegende Erfindung betrifft ein Verfahren zur Herstellung von Galactooligosacchariden (GOS) oder einer Galactooligosaccharid-haltigen Zusammensetzung, bei dem man eine von *L. bulgaricus* (*L. delbrueckii spp. bulgaricus*) abgeleitete beta-Galactosidase (auch Lactase [EC3.2.1.23]) bei einer Temperatur von 37°C oder weniger mit einer Lactose-haltigen Zusammensetzung wie Milch, Puffer oder Molke, z.B. Süßmolke, Sauermolke, Molkenkonzentrat oder Molkenpermeat, inkubiert. Die Erfindung betrifft auch eine Verwendung des Enzyms zur Herstellung von Galactooligosacchariden oder einer Galactooligosaccharid-haltigen Zusammensetzung in einer solchen Lactose-haltigen Zusammensetzung. Ferner wird ein Verfahren zur Herstellung einer Galactooligosaccharid-haltigen Zusammensetzung mit einem definierten Anteil von DP4 Galactooligosacchariden und DP3 Galactooligosacchariden an allen Galactooligosacchariden bereitgestellt, bei dem man eine von *L. bulgaricus* abgeleitete beta-Galactosidase bei einer definierten Temperatur mit einer Lactose-haltigen Flüssigkeit inkubiert. Die Erfindung stellt ferner mit den erfindungsgemäßen Verfahren herstellbare Zusammensetzungen und dabei vorteilhaft einzusetzende, in Caseinpepton-Medium hergestellte beta-Galactosidase und Verfahren zu ihrer Herstellung zur Verfügung.

Als Galactooligosaccharide (GOS) werden Mehrfachzucker bezeichnet, welche aus einer Reihe von Galactosemolekülen und optional einem endständigen Glucosemolekül bestehen. Sie entstehen bei der Spaltung von Lactose (Hydrolyse) und Übertragung der freigesetzten Galactose auf ein anderes Molekül (Transgalactosylierung) durch das Enzym beta-Galactosidase. Zunächst wird ein Lactose-Enzym-Komplex gebildet, aus welchem Glucose freigesetzt wird. Der Galactosyl-Rest verbleibt dabei am aktiven Zentrum des Enzyms. Anschließend können zwei mögliche Reaktionen ablaufen. Wird der Galactosyl-Rest auf ein Wassermolekül übertragen, findet lediglich eine Hydrolyse statt und es entsteht freie Galactose. Ist ein anderes Zuckermolekül der Akzeptor, entstehen GOS. Bei diesem "anderen" Zuckermolekül kann es sich um alle in der Reaktionslösung vorhandenen Zucker handeln (z.B. Lactose oder aus der Hydrolysereaktion entstandene Monosaccharide Glucose und Galactose, aber auch bereits entstandene GOS). Dadurch kommt es zur Kettenverlängerung und es entstehen Oligosaccharide mit einem Polymerisationsgrad (DP) von bis zu neun Monomeren. Die entstandenen Oligosaccharide unterscheiden sich neben der Kettenlänge auch in der Art der glykosidischen Bindung. Weit verbreitet ist z.B. die β-1,6- verknüpfte Allolactose oder auch Galactosyl-Lactosen mit einer 1,6- bzw. 1,3- Verknüpfung.

Im vergangenen Jahrzehnt haben sogenannte funktionelle Lebensmittel, d.h. Lebensmittel mit gesundheitlichem Zusatznutzen, beim Verbraucher zunehmend an Bedeutung gewonnen. Ein Hauptziel von funktionellen Lebensmitteln ist z.B. die positive Beeinflussung des Gastrointestinaltrakts. Dazu gehört auch der Ausbau einer gesunden Darmflora. Hierbei können GOS nützlich sein.

Humane Kolostralmilch enthält ca. 20-25 g/L Oligosaccharide (sog. HMOS), welche maßgeblich für den bifidogenen Effekt der Muttermilch verantwortlich sind. Wenn Mütter nicht ausreichend Milch bereitstellen können, kann Säuglingsergänzungsnahrung auf Kuhmilchbasis die Versorgung mit lebenswichtigen Nährstoffen sicherstellen. Allerdings enthält Kuhmilch deutlich weniger Oligosaccharide (ca. 1/500stel des Gehaltes in humaner Kolostralmilch), sodass es vorteilhaft für Säuglinge ist, wenn Oligosaccharide zugefügt werden. Eine Isolierung von HMOS für kommerzielle Zwecke ist nicht möglich. Aufgrund der strukturellen Ähnlichkeit, werden GOS aus verschiedenen Quellen als wertvoller und geeigneter Ersatz angesehen. Der Einsatz von GOS beschränkt sich daher in Deutschland v.a. auf Säuglings- und Folgenahrung, positive Effekte sind jedoch auch bei Erwachsenen gezeigt.

Die prebiotische Wirkung von GOS wurde bereits in zahlreichen Studien bestätigt. Sie fördern sowohl das Wachstum von Bifidobakterien als auch von Lactobazillen und hemmen pathogene Keime. Durch Zusatz von GOS gleichen sich die Anzahl und die Art der kurzkettigen Fettsäuren von nicht gestillten (Flaschen-) Säuglingen denen der gestillten Säuglinge an. Auswirkungen auf die Stuhlfrequenz und -konsistenz wurden ebenfalls von zahlreichen Autoren beschrieben. Zudem gab es positive Effekte im Hinblick auf Atemwegsinfektionen und eine verbesserte Mineralstoffaufnahme.

Beta-Galactosidase wird von Pilzen, Hefen, Bakterien und Archaebakterien synthetisiert. Es wurde eine Vielzahl von Enzymen auf ihre Eignung zur GOS-Synthese untersucht, z.B. aus *Sulfolobus solfataricus, Pyrococcus furiosuaus, Bacillus circulans, Bifidobacterium infantis, Aspergillus oryzae, Penicillium expansum* und *Kluyveromyces lactis.* Auch Enzyme verschiedener Spezies von Lactobazillen (*L. acidophilus, L. reuterii, L. sakei, L. pentosus, L. plantarum*) wurden auf ihre Eignung zur GOS-Synthese getestet. Bei einer Lactosekonzentration von ca. 20 % lag die Ausbeute zwischen ca. 10 - 50 % bei einem Lactosehydrolysegrad von ca. 25 - 94 % (IQBAL, S. et al. (2010), Carbohydrate Research, Vol. 345, No. 10, 1408-1416; IQBAL, S. et al. (2011) Journal of Agricultural and Food Chemistry, Vol. 59, No. 8, 3803-3811; MAISCHBERGER, T. et al. (2010) Biotechnology Journal, Vol. 5, No. 8, 838-847; MAISCHBERGER, T. et al. (2008), Carbohydrate Research, Vol. 343, 2140-2147; SPLECHTNA, B. et al. (2006) Journal of Agricultural and Food Chemistry, Vol. 54, No. 14, 4999-5006; SPLECHTNA, B., et al. (2007), Journal of Agricultural and Food Chemistry, Vol. 55, No. 16, 6772-6777; SPLECHTNA, B. et al., 2007 Biotechnology Journal 2, 480-485; NGUYEN, T. T. et al. (2012), Journal of Agricultural and Food Chemistry, Vol. 60, 1713-1721; NGUYEN, T.H.et al. (2007) FEMS Microbiology Letters, Vol. 269, No. 1, 136-144; SONG et al., 2013 (Korean J. Food Sci. An. Vol. 33, No. 5, 565-571; VASILJEVIC, T. & JELEN, P. (2003), Lait, Vol. 83, 453-467 und ZHANG, H. et al. (2013), European Food Research and Technology, Vol. 236, 817-826).

Im Allgemeinen wurde die GOS-Synthese in Puffer getestet. Weitaus weniger Publikationen gibt es bei Verwendung anderer Medien wie Milch, Molke und Molkenpermeat. Es ist bekannt, dass einzelne Milchsalze und Proteine die beta-Galactosidase- Aktivität und/oder Stabilität (z.B. Chang und Mahoney 1989, Journal of Dairy Research, Vol. 56, No. 1, 117-127) beeinflussen können, wobei dieser Einfluss von der jeweiligen Enzymquelle abhängt und sowohl positiv (z.B. Flores, M. V., Ertola, R. J., & Voget, C. E. (1996), LWT - Food Science and Technology, 29(5-6), 503-506), als auch negativ (z.B. Greenberg und Mahoney, 1984, Food Chemistry, Vol. 15, No. 4, 307-313) sein kann. Ebenso kann es zu synergistischen Effekten kommen (z.B. Montilla, A., Corzo, N., & Olano, A. (2012), Milchwissenschaft, 67(1), 14-18).

In US 4,944,952 z.B. sind bei Einsatz einer beta-Galactosidase aus *L. bulgaricus* in Milch mit 4,5 % Lactose hohe Ausbeuten (ca. 37 % inkl. GOS-Disaccharide) an GOS beim Temperaturoptimum der verwendeten beta-Galactosidase bei 40°C beschrieben. Vasilijevic und Jelen (2003), Lait 83:453-467, erreichten in Magermilch mit einer Lactosekonzentration von 5 % eine GOS-Ausbeute von ca. 10 % des Gesamtzuckers nach 2 h. Sie verwendeten einen Rohextrakt von *L. bulgaricus* (Stamm ATCC 11842) und setzten 200 U Enzym/ g Lactose ein (pH 6,8, T 50°C).

In der Molkereiindustrie in Deutschland fallen jährlich ca. 12 Mio. t Molke (davon 73 % Süßmolke) an. Es besteht großer Bedarf, dieses Nebenprodukt als preisgünstigen Rohstoff zu nutzen und hochwertige Produkte daraus herzustellen, die z.B. GOS umfassen.

Splechtna et al. (2007), Biotechnology Journal 2:480-485 zeigen die Herstellung von GOS mit Hilfe eines Rohextraktes aus *L. reuterii* mit beta-Galactosidaseaktivität in konzentriertem Molkenpermeat bei einer Lactosekonzentration von ca. 20 %. Versuche mit niedrigen Lactosekonzentrationen werden nicht beschrieben.

Fischer und Kleinschmidt (2015), International Dairy Journal 48:15-22, beschreiben die Möglichkeit der Verwendung von Süß- und Sauermolke für die Herstellung von GOS bei einer Lactosekonzentration von < 5% mit aus *K. lactis* (opti-lactase LX2, optiferm GmbH) bzw. *A. oryzae* (opti-lactase P, optiferm GmbH) isolierten beta-Galactosidasen. Die GOS-Ausbeute lag bei ca. 4 bzw. 10 % des Gesamtzuckers, also relativ gering.

Dem gegenüber lösten die Erfinder das Problem, ein verbessertes Verfahren zur Herstellung von GOS oder GOS-haltigen Zusammensetzungen in einer Lactose-haltigen Zusammensetzung wie Milch, Puffer oder Molke bereitzustellen, bei dem z.B. Molkeprodukte wie Süß- oder Sauermolke umgesetzt werden können und bei dem eine hohe Ausbeute an GOS erreicht wird. Ferner wird durch die Erfindung ein Verfahren zur Herstellung einer Galactooligosaccharid-haltigen Zusammensetzung mit einem definierten Anteil von DP4 Galactooligosacchariden und DP3 Galactooligosacchariden an allen Galactooligosacchariden bereitgestellt.

Das Problem wird durch den Gegenstand der Ansprüche gelöst. Insbesondere stellt die Erfindung ein Verfahren zur Herstellung von GOS oder GOS-haltigen Zusammensetzungen in einer Lactose-haltigen Zusammensetzung wie Milch, Puffer oder Molke bereit, bei dem man eine von *L. bulgaricus* abgeleitete beta-Galactosidase mit Molke inkubiert, bevorzugt bei einer Temperatur von 37°C oder weniger. Im Rahmen der Erfindung ist die Lactose-haltige Zusammensetzung bevorzugt Molke.

Ebenso wird die Verwendung einer von *L. bulgaricus* abgeleiteten beta-Galactosidase zur Herstellung von Galactooligosacchariden oder einer Galactooligosaccharid-haltigen Zusammensetzung in einer Lactose-haltigen Zusammensetzung wie Milch, Puffer oder Molke, bevorzugt bei einer Temperatur von 37°C oder weniger, zur Verfügung gestellt.

Die Erfinder stellten überraschenderweise fest, dass zum einen die beta-Galactosidase von *L. bulgaricus,* insbesondere aus dem Stamm LB4, besonders geeignet zur GOS-Herstellung in Lactose-haltigen Zusammensetzungen wie Milch, Puffer oder Molke normaler Lactosekonzentration ist, also z.B. in Süß- oder Sauermolke ohne Zusatz von Lactose oder ohne Aufkonzentrierung.

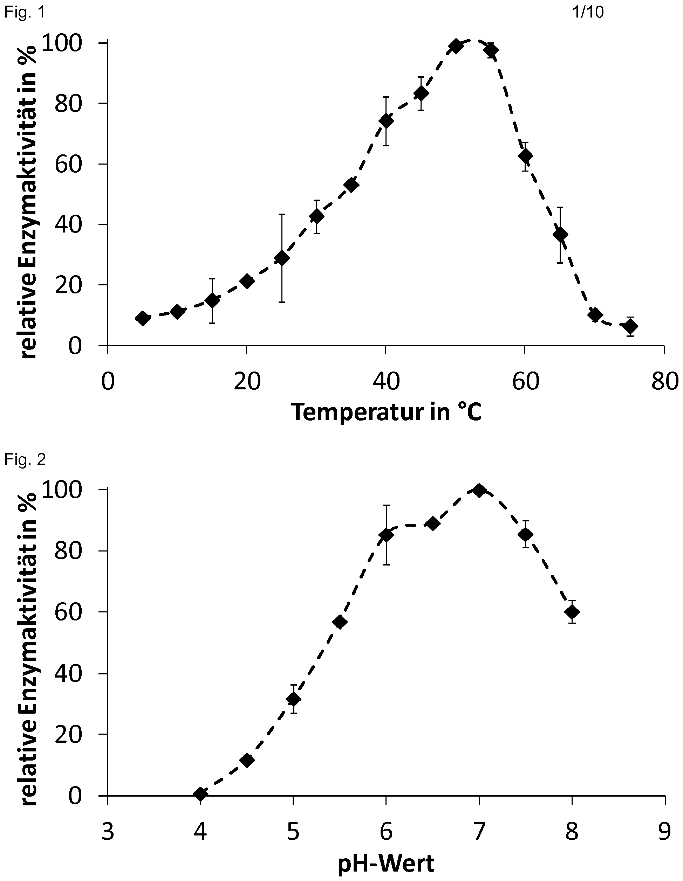

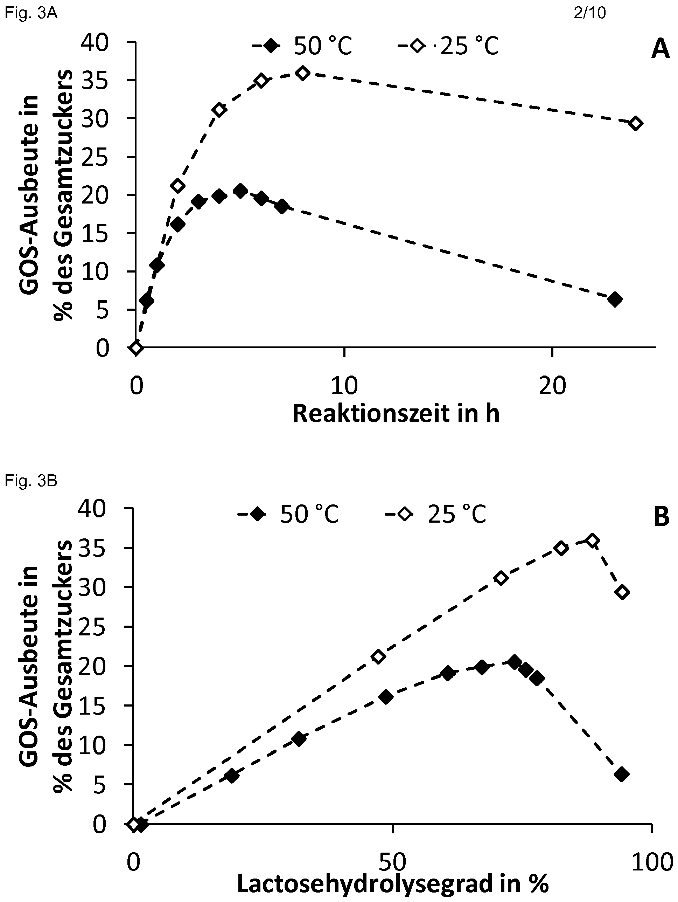

Die Erfinder stellten ferner überraschenderweise fest, dass es, insbesondere zur Optimierung der Ausbeute, günstig ist, die Herstellung von GOS nicht beim Temperaturoptimum des beta-Galactosidase-Enzyms aus *L. bulgaricus* bei 50°C durchzuführen (Fig. 1), das standardgemäß durch Bestimmung der enzymatischen Aktivität bei Spaltung des Lactose-ähnlichen Substrats o-NPG (ortho-Nitrophenyl-beta-D-Galactopyranosid) bestimmt wird, sondern stattdessen eine niedrigere Temperatur von 37°C oder weniger, bevorzugt 30°C oder weniger, zu wählen, bei der die Herstellung von GOS optimal stattfindet (z.B. Fig. 3).

Ferner wurde im Rahmen der Erfindung überraschend ein Zusammenhang zwischen der gewählten Temperatur und dem Polymerisationsgrad der hergestellten GOS gefunden. Erfindungsgemäß kann also die Durchführung des Herstellungsverfahrens in einem definierten Temperaturbereich nicht nur die Menge an GOS beeinflussen, sondern auch die Zusammensetzung der hergestellten GOS kann dadurch gezielt beeinflusst werden.

Damit stellt die Erfindung ein Verfahren zur Herstellung einer Galactooligosaccharid-haltigen Zusammensetzung mit einem definierten Anteil von DP4 Galactooligosacchariden und DP3 Galactooligosacchariden sowie optional auch DP2 Galactooligosacchariden an allen Galactooligosacchariden zur Verfügung, bei dem man eine von *L. bulgaricus* abgeleitete beta-Galactosidase mit einer Lactose-haltigen Flüssigkeit ausgewählt aus der Gruppe umfassend Molke, Milch oder Puffer, bevorzugt Molke, inkubiert, wobei man
a) die Lactose-haltige Flüssigkeit mit der beta-Galactosidase bei einer Temperatur von 10°C oder weniger inkubiert und der Anteil von DP4 Galactooligosacchariden 1 % oder weniger, bevorzugt 0,6 % oder weniger, und der Anteil von DP3 Galactooligosacchariden 22 % oder weniger, bevorzugt 20 % oder weniger beträgt;
b) die Lactose-haltige Flüssigkeit mit der beta-Galactosidase bei einer Temperatur von mehr als 10°C bis 20°C inkubiert und der Anteil von DP4 Galactooligosacchariden 0,5-2 %, bevorzugt 0,6-1,4 % beträgt und der Anteil von DP3 Galactooligosacchariden 18-37 %, bevorzugt 19,8 bis 34,5 % beträgt;
c) die Lactose-haltige Flüssigkeit mit der beta-Galactosidase bei einer Temperatur von mehr als 20°C bis 30°C inkubiert und der Anteil von DP4 Galactooligosacchariden 1-4 %, bevorzugt 1,4-3,4 % beträgt und der Anteil von DP3 Galactooligosacchariden 33-51 %, bevorzugt 34,5-48,9 % beträgt;
d) die Lactose-haltige Flüssigkeit mit der beta-Galactosidase bei einer Temperatur von mehr als 30°C bis 40°C inkubiert und der Anteil von DP4 Galactooligosacchariden 3-4,1 %, bevorzugt 3,4-3,7 % beträgt und der Anteil von DP3 Galactooligosacchariden 46-57 %, bevorzugt 48,9-55,2 % beträgt;
e) die Lactose-haltige Flüssigkeit mit der beta-Galactosidase bei einer Temperatur von mehr als 40°C bis 50°C inkubiert und der Anteil von DP4 Galactooligosacchariden 3,5-5,5 %, bevorzugt 3,7-5,2 % beträgt und der Anteil von DP3 Galactooligosacchariden 53-65 %, bevorzugt 55,2-62,8 % beträgt; oder
f) die Lactose-haltige Flüssigkeit mit der beta-Galactosidase bei einer Temperatur von mehr als 50°C bis 60°C inkubiert und der Anteil von DP4 Galactooligosacchariden mehr als 5 %, bevorzugt mehr als 5,2 % beträgt und der Anteil von DP3 Galactooligosacchariden mehr als 60 %, bevorzugt mehr als 62,8 % beträgt.

%-Angaben beziehen sich im Rahmen der Erfindung immer auf Masse-%, sofern nicht ausdrücklich anders erwähnt. Galactooligosaccharide mit einem höheren Polymerisationsgrad kommen jeweils in einem Anteil von unter 5%, bevorzugt unter 1% vor. Sie sind üblicherweise nicht in nachweisbaren Mengen enthalten. Somit sind die weiteren Galactooligosaccharide (also ad 100%) im Rahmen der Erfindung im Allgemeinen DP2 Galactooligosaccharide.

Die Erfinder haben festgestellt, dass bei einer Temperatur der Inkubation von mehr als 30 - 37°C eine GOS-haltige Zusammensetzung hergestellt werden kann, welche einen Anteil von 3-4,1 %, bevorzugt 3,4-3,7 % DP4 Galactooligosacchariden und 46-57 %, bevorzugt 48,9-55,2 % DP3 Galactooligosacchariden an allen Galactooligosacchariden aufweist, und wobei der Anteil der GOS an dem Gesamtzucker mindestens 15 %, bevorzugt, mindestens 16 %, mindestens 17 %, mindestens 18 %, mindestens 20 % oder mindestens 22 % beträgt.

In einer Ausführungsform der Erfindung beträgt die Temperatur bei der Inkubation 30°C oder weniger, z.B. Raumtemperatur (etwa 25°C) oder weniger. Bevorzugt liegt in dem hergestellten Produkt der Anteil der GOS an dem Gesamtzucker bei mindestens 13 %, mindestens 14 %, mindestens 15 %, mindestens 16 %, mindestens 17 %, mindestens 20 % oder mindestens 22 %.

Die Erfinder haben festgestellt, dass bei dieser Temperatur der Inkubation eine GOS-haltige Zusammensetzung hergestellt werden kann, welche einen Anteil von 4 % oder weniger, bevorzugt 3,4 % oder weniger DP4 Galactooligosacchariden und 51 % oder weniger, bevorzugt 48,9 % oder weniger DP3 Galactooligosacchariden an allen Galactooligosacchariden aufweist, und wobei der Anteil der GOS an dem Gesamtzucker mindestens 13 %, bevorzugt, mindestens 14 % beträgt. Bevorzugt liegt der Anteil der GOS an dem Gesamtzucker bei mindestens 15 %, mindestens 16 %, mindestens 17 %, mindestens 20 % oder mindestens 22 %.

In einer Ausführungsform der Erfindung beträgt die Temperatur bei der Inkubation 20°C oder weniger. Bevorzugt liegt in dem hergestellten Produkt der Anteil der GOS an dem Gesamtzucker bei mindestens 15 %, mindestens 16 %, mindestens 17 %, mindestens 20 % oder mindestens 22 %.

Die Erfinder haben festgestellt, dass bei dieser Temperatur der Inkubation eine GOS-haltige Zusammensetzung hergestellt werden kann, welche einen Anteil von 2 % oder weniger, bevorzugt 1,4 % oder weniger DP4 Galactooligosacchariden und 37 % oder weniger, bevorzugt 34,5 % oder weniger DP3 Galactooligosacchariden an allen Galactooligosacchariden aufweist, wobei der Anteil der GOS an dem Gesamtzucker mindestens 15 %, bevorzugt, mindestens 16 %, mindestens 17 %, mindestens 20 % oder mindestens 22 % beträgt.

In einer Ausführungsform der Erfindung beträgt die Temperatur bei der Inkubation 10°C oder weniger, insbesondere 0-10°C oder z.B. ca. 4-8°C. Bevorzugt liegt in dem hergestellten Produkt der Anteil der GOS an dem Gesamtzucker bei mindestens 17 %, mindestens 18 %, mindestens 19%, mindestens 20 % oder mindestens 22 %.

Bevorzugt wird bei einer Temperatur der Inkubation von 10°C oder weniger eine GOS-haltige Zusammensetzung hergestellt, welche einen Anteil von 1 % oder weniger, bevorzugt 0,6 % oder weniger DP4 Galactooligosacchariden und 22 % oder weniger, bevorzugt 20 % oder weniger DP3 Galactooligosacchariden an allen Galactooligosacchariden aufweist, wobei der Anteil der GOS an dem Gesamtzucker mindestens 17 %, mindestens 18 %, mindestens 19%, bevorzugt mindestens 20 % oder mindestens 22 % beträgt. Die Erfindung stellt somit ein Verfahren zur Herstellung von GOS-haltigen Zusammensetzungen mit unterschiedlichem Polymerisationsgrad zur Verfügung, insbesondere ein Verfahren zur Modulation des Polymerisationsgrades von GOS, bei dem beta-Galactosidase aus *L. bulgaricus,* z.B. aus dem Stamm LB4, bei verschiedenen Temperaturen mit einer Lactose-haltigen Zusammensetzung ausgewählt aus der Gruppe umfassend Molke, Milch und Puffer, bevorzugt Molke, inkubiert wird.

Die Lactose-haltige Zusammensetzung kann z.B. Milch sein, wobei Rohmilch, Milch mit einem Fettgehalt von mehr als 1,8 %, insbesondere Vollmilch mit einem Fettgehalt von 3,5-3,8 %), oder fettarme Milch (mit einem Fettgehalt von 1,5-1,8 %) oder Milch mit einem Fettgehalt unter 1,5 %, z.B. Magermilch mit 0,1-0,5 % Fett, eingesetzt werden kann.

Die Lactose-haltige Flüssigkeit kann auch ein Puffer sein, insbesondere gepuffert auf einen pH-Wert von 4-8, bevorzugt pH 5-7 oder ca. pH 6-6,5. Die Puffer sind für die gewünschte Umsetzung geeignet. Es können z.B. folgende Puffer eingesetzt werden: Natrium-Phosphat-Puffer, Kalium-Phosphat-Puffer, Natrium/Kalium-Phosphat-Puffer, Citrat-Puffer, Acetat-Puffer.

Bevorzugt wird jedoch Molke als Edukt eingesetzt. Je nach Art der Casein-Fällung werden verschiedene Arten von Molke unterschieden. Bei der Käseherstellung erfolgt die Fällung mit Lab-Enzymen. Die daraus resultierende Molke wird Süßmolke oder auch Labmolke genannt. Bei der Frischkäse- und Quarkproduktion wird mit Milchsäurebakterien gefällt, wodurch die sogenannte Sauermolke entsteht. Bei der Casein-Herstellung wiederum wird durch die direkte Zugabe von Mineralsäuren (z.B. HCl) gefällt. Dabei entsteht eine besondere Art von Sauermolke, die Caseinmolke. Während der Trockenmasse- und Lactosegehalt bei allen drei Molkenarten ähnlich ist, bestehen zum Teil erhebliche Unterschiede bei Fett-, Asche- und Proteingehalt. Mit 0,75 % enthält Süßmolke mehr als doppelt so viel Protein wie Sauermolke und um 1/3 mehr als Caseinmolke. Der pH-Wert von Süßmolke liegt bei ca. 6,1, der pH-Wert von Sauermolke bei etwa 4,6 und der pH-Wert von Caseinmolke bei ca. 4,4.

Molkenpermeat ist ein Nebenerzeugnis, das durch Ultrafiltration aus flüssiger Molke gewonnen werden kann. Dabei verbleiben die großmolekularen Molkenproteine im Retentat, währen das Permeat Lactose und weitere kleinere Molkenbestandteile umfasst. Es kann Süßmolkenpermeat oder Sauermolkenpermeat hergestellt werden, welches einen sauren pH-Wert aufweist.

Erfindungsgemäß kann die Molke ausgewählt sein aus der Gruppe umfassend Sauermolke, Süßmolke, Caseinmolke und Molkenpermeat, bevorzugt Süßmolke. Als Substrat für die GOS-Synthese können also verschiedene Molkearten (Süßmolke, Sauermolke, Caseinmolke) dienen.

Die Molke kann eine Trockenmasse (TM) von ca. 6,5 % (entsprechend frischer Molke) bis ca. 60 % (nach dem Eindampfen etwa in einer Molkerei) aufweisen. Selbstverständlich ist es auch möglich, die Molke auf Basis von Pulver (Molkenpulver, z.B. Süßmolkenpulver, Molkenpermeatpulver usw.) herzustellen, indem das Pulver z.B. in Wasser gelöst wird.

Der Molke kann Lactose hinzugefügt werden, um die Konzentration des Ausgangsprodukts zu erhöhen, bevorzugt ist dies jedoch nicht der Fall. Durch Eindampfen oder durch Lösung von Pulver in einer geringeren Menge an Wasser kann die Konzentration der Molke auch erhöht werden, z.B. auf die 1,1-10- fache Konzentration, insbesondere auf die 2-4- fache Konzentration. Es können also auch Molkenkonzentrate verwendet werden, z.B. Molkenkonzentrate aus Sauermolke, Süßmolke, Caseinmolke oder Molkenpermeat, wobei Molkenkonzentrat aus Süßmolke wirtschaftlich am relevantesten ist. Erfindungsgemäß ist dies jedoch nicht nötig. Molkenkonzentrate können z.B. vor Inkubation mit der beta-Galactosidase wieder teilweise, z.B. auf die 2-4- fache Konzentration, oder komplett auf eine für unkonzentrierte Molke typische Konzentration (z.B. etwa 6-7 % Trockenmasse) verdünnt werden, z.B. mit Wasser. Im Allgemeinen ist bei der Inkubation mit der beta-Galaktosidase weder die Lactosekonzentration erhöht noch die Molkenkonzentration insgesamt.

Die Lactosekonzentration in der Molke liegt somit bei ca. 20-400 g/L, z.B. bei 20-350 g/L, 25-100 g/L oder bevorzugt 30-60 g/L, z.B. für Süßmolke ca. 40-55 g/L, insbesondere ca. 47-48 g/L, bei Sauermolke ca. 35-49 g/L, insbesondere ca. 42 g/L oder bei Caseinmolke ca. 42-56 g/L, insbesondere ca. 49 g/L (bei ca. 6 % TM). In Molkenpermeat sind typische Lactosekonzentrationen ähnlich, z.B. 35-50 g/L. Die Lactosekonzentration in Molkenkonzentrat kann z.B. bei 250-400 g/L liegen, bevorzugt bei 300-350 g/L. Bevorzugt liegt die Lactosekonzentration unter 60 g/L.

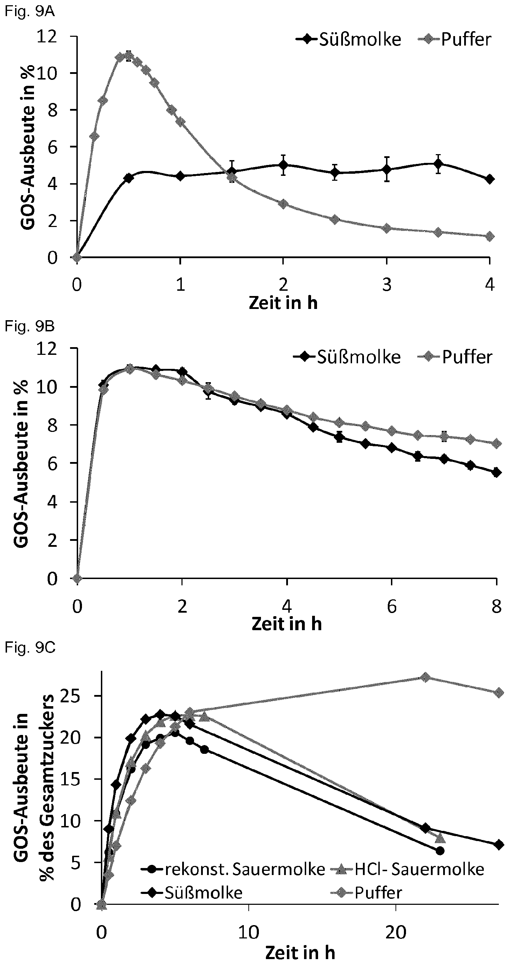

Vorteilhafterweise kann man mit der erfindungsgemäßen beta-Galactosidase aus *L. bulgaricus* in allen Arten der Molke gute Ausbeuten bei der Herstellung von GOS erreichen, die die Ausbeuten mit Enzymen aus *K*. *lactis* und *A. oryzae* überschreiten (Fig. 9).

Da das pH-Optimum der eingesetzten beta-Galactosidasen bei pH 6-7 liegt (Fig. 2), kann Süßmolke oder Süßmolkenpermeat ohne Einstellung des pH-Werts eingesetzt werden. Weist das eingesetzte Molkenprodukt einen pH von unter 6 auf, so wird der pH-Wert bevorzugt vor der Inkubation mit dem Enzym auf pH 6-7, bevorzugt 6,5-7, eingestellt. Selbstverständlich kann auch der pH von Süßmolke auf 6,5-7 eingestellt werden, es ist jedoch vom Verfahrensablauf her im Allgemeinen effizienter, diesen Schritt wegzulassen.

Die Molke ist bevorzugt durch Abtrennung von Caseinteilchen (Käsestaub) vorbehandelt z.B. durch Vibrations/Rotationssiebe und/oder die Abscheidung von Fett über Zentrifugalseparatoren zur Gewinnung von Molkenrahm. Dabei werden bevorzugt beide Schritte durchgeführt. Dies findet typischerweise vor einer Keimreduktion statt.

Die Molke ist bevorzugt vorbehandelt, um das Risiko einer mikrobiellen Kontamination zu senken. Eine Keimreduzierung ist durch Pasteurisierung und Wärmebehandlung (z.B. Kurzzeiterhitzung, etwa 71-74°C/≥15 s) möglich. Im Prinzip ist eine Keimreduzierung auch durch Mikrofiltration oder Baktofugierung möglich, wird aber praktisch eher weniger umgesetzt.

Besonders gute Ergebnisse wurden erfindungsgemäß mit einer beta-Galactosidase von Stamm LB4 erzielt, sodass die eingesetzte beta-Galactosidase bevorzugt von diesem Stamm abgeleitet ist. Abgeleitet bedeutet, dass die beta-Galactosidase aus einer Kultur von *L. bulgaricus,* z.B. von LB4, isoliert sein kann. Dabei kann es sich auch um einen Rohextrakt handeln, der mit einem Verfahren enthalten wird, Schritte umfassend, bei denen man die Zellen lysiert und eine unlösliche Fraktion, welche Zellmembranen umfasst, z.B. durch Zentrifugation abtrennt. Da das Enzym intrazellulär ist, werden üblicherweise keine lebenden Bakterien zur Herstellung der GOS eingesetzt. Tote Zellen können verwendet werden, bevorzugt ist das Enzym jedoch isoliert, mindestens in Form eines Rohextrakts. Auch ein im Wesentlichen reines Enzym kann eingesetzt werden. Alternativ kann das Enzym aber auch gentechnisch hergestellt werden, z.B. in *E. coli,* wobei bevorzugt eine aufgereinigte Form des Enzyms eingesetzt wird.

In einer Ausführungsform weist die beta-Galactosidase mindestens 90 %, bevorzugt mindestens 95 % oder mindestens 99 % Sequenzidentität zu beta-Galactosidase aus *L. bulgaricus* Stamm 11842 auf (UniprotKB - Q1G9Z4 (BGAL_LACDA), wobei die beta-Galactosidase bevorzugt 100 % Sequenzidentität dazu aufweist.

Überraschenderweise haben die Erfinder gefunden, dass die beta-Galactosidase von *Lactobacillus bulgaricus* nicht nur in verschiedenen Medien unterschiedlich gut hergestellt wird, sondern sich auch in ihren Eigenschaften bei der Herstellung von GOS, z.B. in Bezug auf das Spektrum an GOS, unterscheidet, je nachdem, in welchem Medium die Bakterien kultiviert wurden, aus denen das Enzym isoliert wurde. Die Erfindung stellt daher auch ein Verfahren zur Modulation des Polymerisationsgrades von GOS zur Verfügung, bei dem die beta-Galactosidase aus *L. bulgaricus,* kultiviert in verschiedenen Medien, hergestellt wird. Insbesondere ist Gegenstand der Erfindung ein Verfahren zur Steigerung des Polymerisationsgrades von GOS, bei dem die beta-Galactosidase aus *L. bulgaricus,* kultiviert in Caseinpeptide enthaltendem Medium, insbesondere Caseinpepton-Medium, hergestellt wird, bevorzugt in Caseinpepton-MRS-Medium. Das Medium umfasst bevorzugt ca. 5-15 g/L Caseinpepton, insbesondere etwa 10 g/L Caseinpepton. Bevorzugt ist kein Fleischpepton und kein Fleischextrakt enthalten. Das Medium umfasst bevorzugt ferner Lactose, z.B. 10-30 g/L, bevorzugt ca. 20 g/L.

Bevorzugt wurde aus dem so kultivierten *L. bulgaricus* ein Rohextrakt des Enzyms durch Homogenisierung und Abzentrifugierung hergestellt. Das Enzym kann sterilisiert sein und z.B. mit Glycerol, z.B. 50 % Glycerol stabilisiert werden.

### Die Erfindung stellt damit auch ein Verfahren zur Verfügung, bei dem man

a) *L. bulgaricus* (z.B. Stamm LB4) in einem Caseinpeptide enthaltenden Medium, bevorzugt, einem Caseinpepton-Medium, kultiviert und beta-Galactosidase zumindest teilweise isoliert;
b) eine Lactose-haltige Flüssigkeit ausgewählt aus der Gruppe umfassend Molke, Milch oder Puffer, mit der zumindest teilweise isolierten beta-Galactosidase inkubiert; und
c) optional Galactooligosaccharide aufreinigt.

Bevorzugt ist die Lactose-haltige Flüssigkeit Molke. Optional wird nach Schritt b die beta-Galactosidase inaktiviert.

### Damit stellt die Erfindung ein Verfahren zur Verfügung, bei dem man

a) *L. bulgaricus* in einem Caseinpeptide enthaltenden Medium, bevorzugt, in einem Caseinpepton-Medium, kultiviert und beta-Galactosidase isoliert;
b) eine Lactose-haltige Flüssigkeit ausgewählt aus der Gruppe umfassend Molke, Milch oder Puffer mit der isolierten beta-Galactosidase inkubiert;
c) die Inkubation stoppt, wenn ein Hydrolysegrad der Lactose von 60-85 % erreicht ist, bevorzugt durch Hitzeinaktivierung, und
d) optional Galactooligosaccharide aufreinigt und/oder Zutaten hinzufügt. Ferner kann das Verfahrensprodukt getrocknet oder verdünnt werden.

Offenbart ist auch ein Verfahren zur Herstellung einer beta-Galactosidase aus *L. bulgaricus,* bei dem man *L. bulgaricus,* bevorzugt aus Stamm LB4, in einem Caseinpepton-Medium inkubiert und eine beta-Galactosidase zumindest teilweise isoliert, und eine mit diesem Verfahren herstellbare beta-Galactosidase aus *L. bulgaricus,* bevorzugt aus Stamm LB4.

Die Erfinder haben festgestellt, dass die Konzentration an hergestellten GOS am höchsten ist, wenn man die Inkubation stoppt, wenn ein Hydrolysegrad der Lactose von 60-85 %, bevorzugt, 70-80 % erreicht ist. Bevorzugt findet dies statt, indem das Enzym durch Hitze inaktiviert wird. Dazu können z.B. die Verfahren der Kurzzeiterhitzung, Hocherhitzung, Ultrahocherhitzung oder Dauererhitzung eingesetzt werden. Bei allen diesen Verfahren wird, obwohl die beta-Galactosidase eine hohe Hitzestabilität aufweist, eine Keimabtötung unter Bedingungen erreicht, die die beta-Galactosidase inaktivieren und das Produkt durch Proteindenaturierung und Thiamin- oder Lysin-Schädigung nicht zu stark schädigen. Dabei können die Ziele eingehalten werden, dass eine Denaturierung von beta-Lactoglobulin von weniger als 5 % verursacht wird, eine Farbveränderung oder HMF-Bildung nicht signifikant stattfindet und Thiamin und Lysin erhalten bleiben (vgl. Beispielteil). Zum Beispiel wird die beta-Galactosidase von *L. bulgaricus,* z.B. von LB4 mit einer Hitzeinaktivierung bei 70-100°C für 0,4 s bis 3 min inaktiviert, bevorzugt 70-95°C bei 0,5 s bis 2 min, z.B. ca. 70°C/ca. 2 min bevorzugt bei 80°C/10 s, oder ca. 95°C/ca. 0,5 s.

Die erfindungsgemäß hergestellte GOS-haltige hergestellte Zusammensetzung kann, optional nach weiterer Modifikation, z.B. Hinzufügung weiterer Zutaten wie Aroma, Fruchtextrakt, Süßungsmittel, Gewürze, Verdickungsmittel (z.B. Xanthan), Salz und/oder Schokolade, direkt als Lebensmittel oder Futtermittel, bevorzugt als Lebensmittel eingesetzt werden. Alternativ kann sie getrocknet und in dieser Form oder nach Lösung in Wasser oder einem anderen Lösungsmittel als Lebensmittel oder Futtermittel, bevorzugt als Lebensmittel eingesetzt werden.

Die GOS-haltige hergestellte Zusammensetzung kann z.B. ausgewählt sein aus der Gruppe umfassend Säuglingsnahrung, insbesondere Milchpulver, Kleinkindnahrung, Diätnahrung, Pharmazeutika, Nutrazeutika ("functional foods"), Brei, Milchprodukte, Joghurt, Milchgetränke, Molkengetränke, Energy-Drinks, Fruchtsaft, Dessert, Snacks, Schokolade, Kekse, und Tierfutter.

Gegenstand der Erfindung ist auch eine Zusammensetzung, umfassend Molkenproteine und Galactooligosaccharide in einem Anteil der Galactooligosaccharide an dem Gesamtzucker von mindestens 13 % und, bevorzugt, isolierte beta-Galactosidase abgeleitet aus *L. bulgaricus*. Während der Durchführung der ersten Verfahrensschritte ist die beta-Galactosidase nicht denaturiert. In einer bevorzugten Ausführungsform, z.B. nach Abschluss des Herstellungsverfahrens, ist die beta-Galactosidase denaturiert. Die erfindungsgemäßen Zusammensetzungen umfassen bevorzugt, wenn auch die beta-Galactosidase denaturiert ist, 10 % oder weniger, insbesondere 1-10 % denaturiertes beta-Lactoglobulin oder weniger als 5-10 % denaturiertes beta-Lactoglobulin.

### Die Zusammensetzung kann dann ausgewählt sein aus der Gruppe umfassend

a) eine Zusammensatzung mit einen Anteil von weniger als 1 % DP4 Galactooligosacchariden und weniger als 25 % DP3 Galactooligosacchariden an allen Galactooligosacchariden, wobei der Anteil der Galactooligosaccharide an dem Gesamtzucker mindestens 18 %, bevorzugt, mindestens 20 % beträgt,
b) eine Zusammensatzung mit einen Anteil von 1-2 % DP4 Galactooligosacchariden und 25-42 % DP3 Galactooligosacchariden an allen Galactooligosacchariden, wobei der Anteil der Galactooligosaccharide an dem Gesamtzucker mindestens 15 %, bevorzugt, mindestens 16 % beträgt,
c) eine Zusammensatzung mit einen Anteil von mehr als 2-5 % DP4 Galactooligosacchariden und mehr als 42-52 % DP3 Galactooligosacchariden an allen Galactooligosacchariden,
d) eine Zusammensatzung mit einen Anteil von mehr als 2-5 % DP4 Galactooligosacchariden und mehr als 52-60 % DP3 Galactooligosacchariden an allen Galactooligosacchariden, und
e) eine Zusammensatzung mit einen Anteil von mindestens 4,5 % DP4 Galactooligosacchariden und mindestens 55 % DP3 Galactooligosacchariden an allen Galactooligosacchariden.

Diese Zusammensetzungen sind mit dem erfindungsgemäßen Verfahren herstellbar, bevorzugt aus Molke.

Als Lebens- oder Futtermittel können erfindungsgemäß hergestellte Zusammensetzungen eine positive Beeinflussung des Gastrointestinaltrakts bewirken, u.a. zum Ausbau einer gesunden Darmflora beitragen. Vor allem bei Säuglingsnahrung, die Muttermilch ersetzen oder ergänzen soll, spielt der bifidogene Effekt eine wichtige Rolle.

Alternativ ist es möglich, die GOS aus der GOS-haltigen Zusammensetzung, z.B. aus der GOS-haltigen Molke zu isolieren, beispielsweise durch Abtrennung der Molkenproteine mittels Membranverfahren (z.B. Ultrafiltration, Diafiltration) oder Hitzefällung, auch kombinierte Hitze-Säurefällung oder Zentrifugation oder eine Kombination der genannten Verfahren. Isolierte GOS können, z.B. in Form von konzentriertem GOS-Sirup (z.B. durch mehrstufige Eindampfung unter Vakuum oder Membranfiltration (z.B. Umkehrosmose)) oder GOS-Pulver/pulverförmigem GOS (z.B. mittels Sprühtrocknung) als Lebensmittel- oder Futtermittel-Zutat genutzt werden. Ebenso ist es möglich, im Anschluss an das erfindungsgemäße Verfahren eine Reduktion des Gesamtzuckers durch gezielte Entfernung von Glucose und/oder Galactose und/oder Lactose zu erreichen. Hierfür eignet sich z.B. eine Größenausschlusschromatographie (im batch- oder simulated moving bed (SMB)- Verfahren), eine Behandlung mit Aktivkohle, Membranfiltration, eine Hefefermentation oder die selektive enzymatische Oxidation von Lactose (z.B. mit Cellobiose-Dehydrogenase) und/oder Glucose (z.B. mit Glucose-Oxidase), optional mit anschließender Abtrennung der entstandenen Säuren (Lactobionsäure bzw. Gluconsäure), z.B. mittels Elektrodialyse oder Ausfällung als Calcium-Salz. Die Reduktion freier Glucose ist ebenso parallel zur GOS-Synthese denkbar.

In einer Ausführungsform umfasst die hergestellte GOS-haltige Zusammensetzung isolierte GOS. In einer Ausführungsform handelt es sich um eine Lactose-freie GOS-haltige Zusammensetzung.

### Legende

**Fig. 1** Temperaturoptimum von beta-Galactosidase aus *Lactobacillus bulgaricus* Stamm LB4. Assaybedingungen: 22 mM o-NPG in 50 mM Na-Phosphat pH 7,0, 10 min.
**Fig. 2** pH-Optimum von beta-Galactosidase aus *Lactobacillus bulgaricus* Stamm LB4. Assaybedingungen: 22 mM o-NPG in 50 mM Na-Phosphat, 30°C, 10 min.
**Fig. 3** Einfluss der Temperatur auf die GOS-Ausbeute in Sauermolke (ca. 39 g/L Lactose, eingestellt auf pH 6,5) mit beta-Galactosidase aus LB4 (30 U/g) in Abhängigkeit von (A) Zeit und (B) Hydrolysegrad.

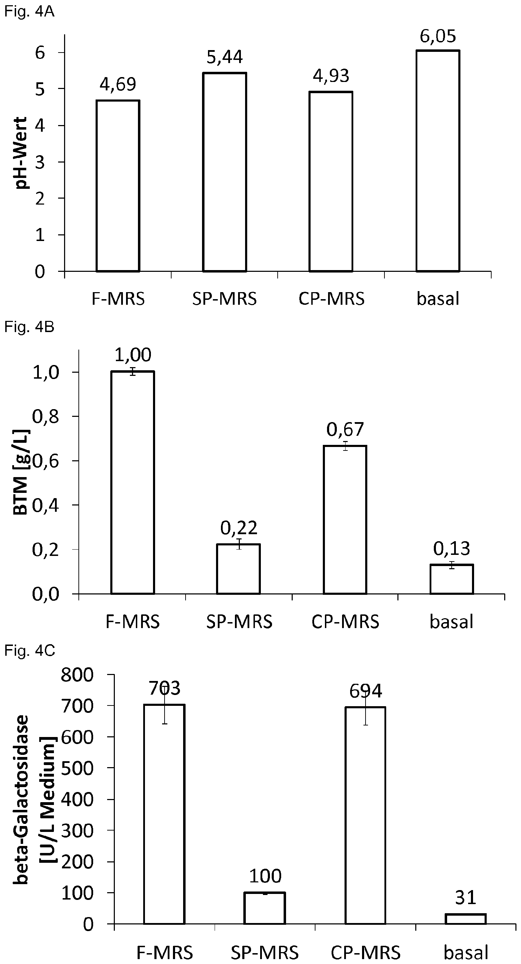

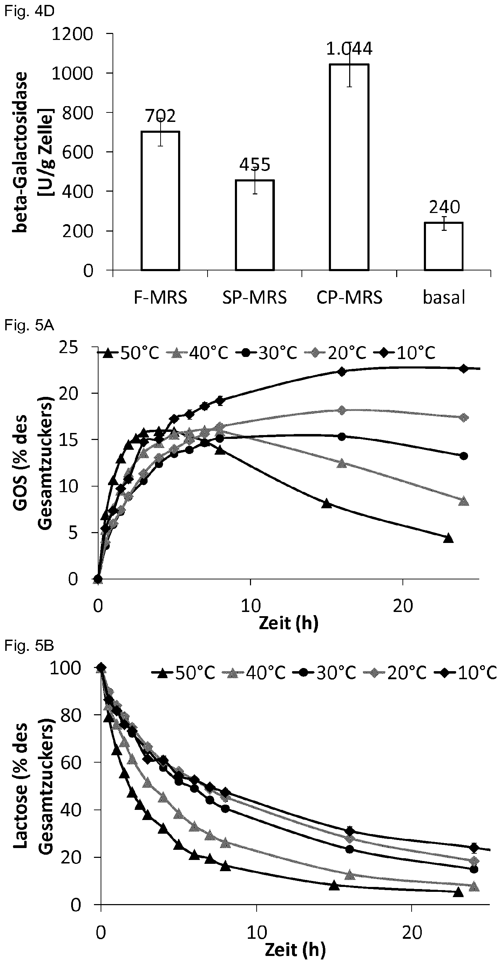

**Fig. 4** Vergleich verschiedener Medien (basales Medium ohne komplexe N-Quelle, nur Ammoniumcitrat (basal), Standard-MRS mit Fleischpepton und Fleischextrakt (F-MRS), Caseinpepton (CP)- und Sojapepton (SP)-MRS) für das Wachstum von *L. bulgaricus* Stamm LB4. Der Versuch erfolgte in Doppelbestimmung an unabhängigen Versuchstagen. A: pH-Wert B: BTM C: Ausbeute an beta-Galactosidase pro Liter Medium D: Ausbeute an beta-Galactosidase pro Gramm Zelle.

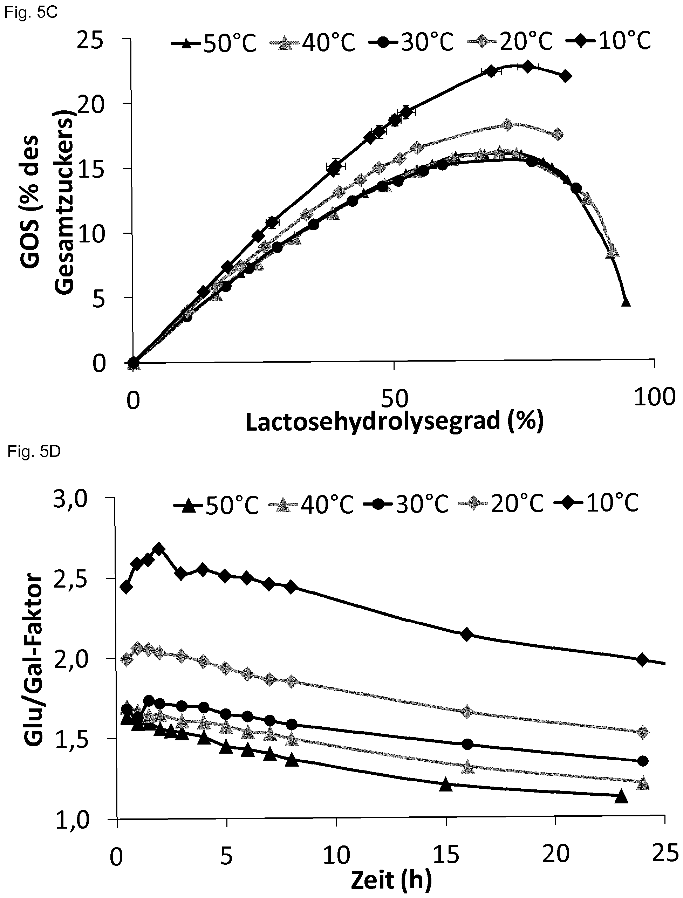

**Fig. 5** GOS-Synthese in Puffer (50 mM Na-Phosphat, pH 6,5) in Abhängigkeit von der Temperatur. A: GOS-Ausbeute (% des Gesamtzuckers) in Abhängigkeit der Reaktionszeit, B: Lactose (% des Gesamtzuckers) in Abhängigkeit der Reaktionszeit, C: GOS-Ausbeute (% des Gesamtzuckers) in Abhängigkeit vom Lactosehydrolysegrad, D: Transgalactosylierungsaktivität gemessen am Glucose/Galactose-Faktor.

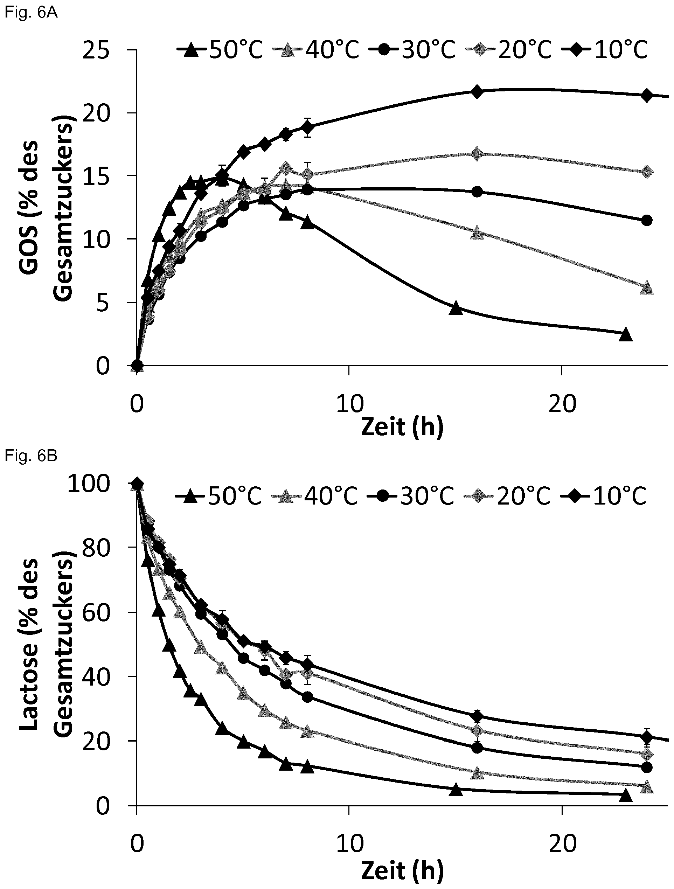

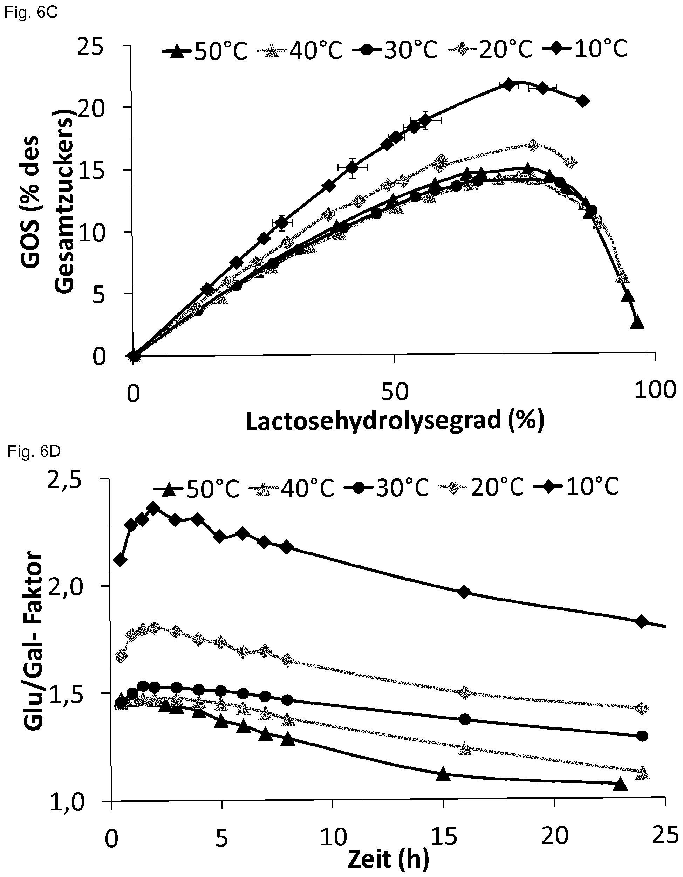

**Fig. 6** GOS-Synthese in Süßmolke (pH eingestellt auf 6,5) in Abhängigkeit von der Temperatur. A: GOS-Ausbeute (% des Gesamtzuckers) in Abhängigkeit der Reaktionszeit, B: Lactose (% des Gesamtzuckers) in Abhängigkeit der Reaktionszeit, C: GOS-Ausbeute (% des Gesamtzuckers) in Abhängigkeit vom Lactosehydrolysegrad, D: Transgalactosylierungsaktivität gemessen am Glucose/Galactose-Faktor.

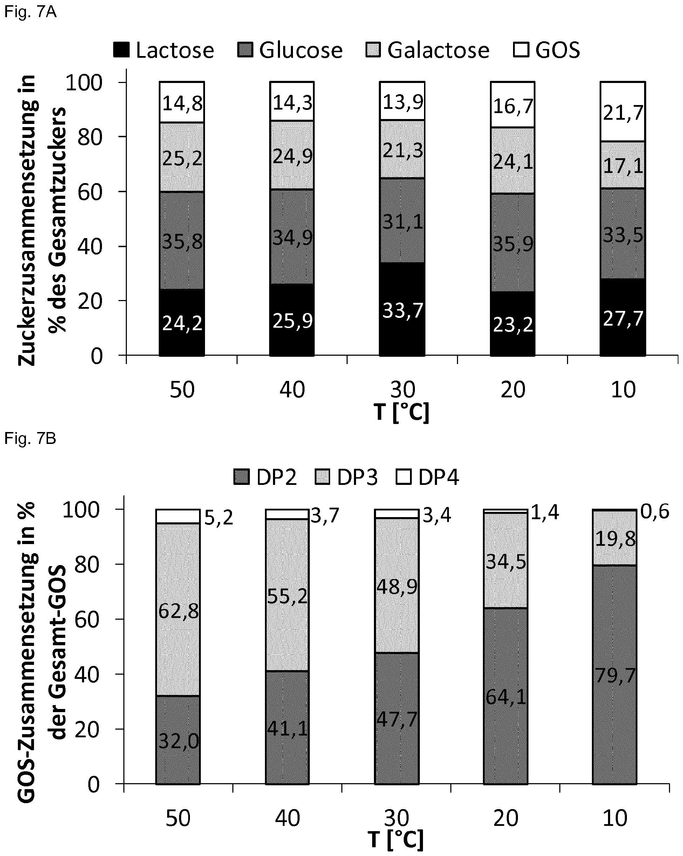

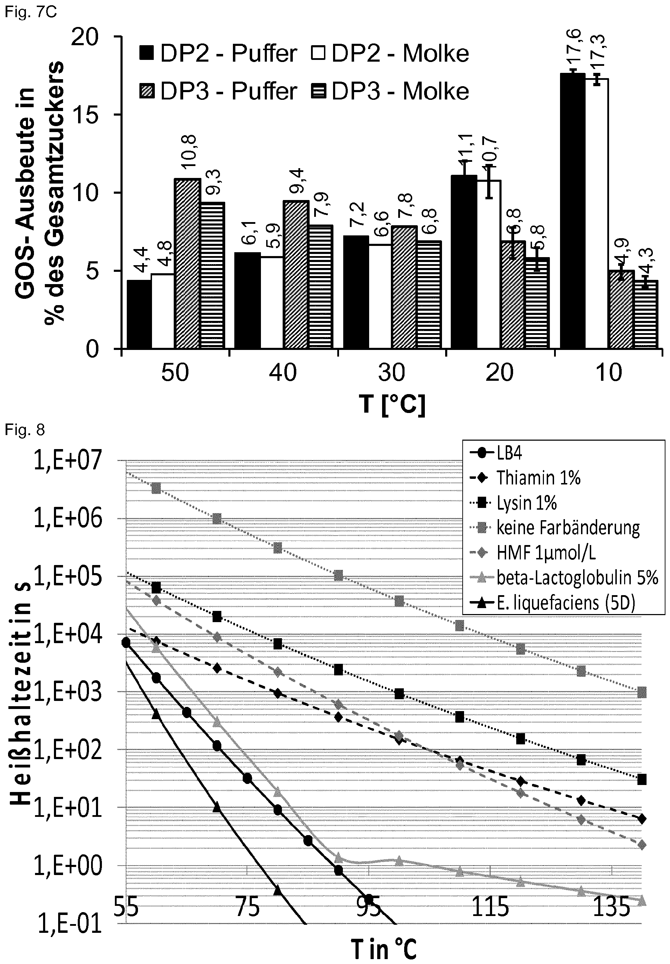

**Fig. 7** A: Zusammensetzung der Zucker nach GOS-Synthese in Molke zum Zeitpunkt der maximalen Ausbeute. B: GOS-Zusammensetzung in Molke zum Zeitpunkt der maximalen Ausbeute in Abhängigkeit der Synthesetemperatur. C: Anteil der GOS-Hauptfraktion (DP2, DP3) am Gesamtzucker in Puffer (50 mM Na-Phosphat, pH 6,5) und Molke zum Zeitpunkt der maximalen Ausbeute in Abhängigkeit der Synthesetemperatur.
**Fig. 8**: Linien gleichen Effekts zur Beurteilung der Enzym- und Keiminaktivierung. Die Temperatur ist gegen die logarithmische Darstellung der Inaktivierungszeit aufgetragen: Es werden die in Milch und Molke relevanten pathogenen Keime (5D- Regime, am Beispiel von *Enterobacter liquefaciens* mit Eₐ = 334 kJ/mol, D_{73,9°C} = 0,56 s), chemische Veränderungen (5 % beta-Lactoglobulindenaturierung (70-90°C: k₀ = 6,90*10³⁸ 1/s, Eₐ = 279,96 kJ/mol, 95-100°C: k₀ = 3,15*10⁵ 1/s, Eₐ = 47,75 kJ/mol), 1% Schädigung an Thiamin (k₀ = 8,57*10⁹ 1/s, Eₐ = 100,8 kJ/mol), 1% Verlust an Lysin (k₀ = 1,95*10¹⁰ 1/s, Eₐ = 109 kJ/mol), keine Farbänderung (F*=1, k₀ = 1,95*10¹⁰ 1/s, Eₐ = 116 kJ/mol), HMF (c = 1 µmol/L, k₀ = 1,62*10¹⁷ 1/s, Eₐ = 139 kJ/mol) sowie die beta-Galactosidase aus dem Stamm LB4 (doppelte Halbwertszeit in 50 mM Na-Phosphat-Puffer pH 6,5) betrachtet.
**Fig. 9****:** Vergleich von GOS-Herstellung in Molke und Puffer (39 g/L Lactose) mit beta-Galactosidase aus *K. lactis* (opti-lactase LX2, optiferm GmbH, A) bzw. *A. oryzae* (opti-lactase P, optiferm GmbH, B) und aus *L. bulgaricus* Stamm LB4 (C) Synthesebedingungen in Puffer: *K*. *lactis:* PEM-Puffer nach FCC IV pH 6,5, 45°C, 50 U/g; *A. oryzae:* dest. Wasser, pH 4,5, 55°C, 50 U/g; *L. bulgaricus* Stamm LB4: 50 mM Na-Phosphatpuffer pH 6,5, 50°C, 30 U/g; Synthese in Molke: Süßmolke 38-39 g/L Lactose, rekonstituierte Sauermolke 36 g/L Lactose, HCl-Sauermolke (mit HCl auf pH 4,6 angesäuerte Magermilch, entspricht sog. Caseinmolke) 42 g/L Lactose; Der pH-Wert der Molken wurden vor der Synthese auf 6,5 (für *K*. *lactis* und *L. bulgaricus*) bzw. 4,5 (für *A*. *oryzae*) eingestellt.

### Beispiele

### Beispiel 1: Kultivierung von Lactobacillus bulgaricus in verschiedenen Medien

### A) Kultivierung

**Tabelle 1: Zusammensetzung verschiedener MRS- Flüssigmedien**

| **Komponente** | **c [g/L]** | | | |
|---|---|---|---|---|
| | **basal** | **MRS (Standard)** | **CP-MRS** | **SP-MRS** |
| Sojapepton (SP) | - | - | - | 10,0 |
| Caseinpepton (CP) | - | - | 10,0 | - |
| Fleischpepton | - | 10,0 | - | - |
| Hefeextrakt | 4,0 | 4,0 | 4,0 | 4,0 |
| Tween 80 | 1,0 | 1,0 | 1,0 | 1,0 |
| Natriumacetat | 5,0 | 5,0 | 5,0 | 5,0 |
| Magnesiumsulfat | 0,2 | 0,2 | 0,2 | 0,2 |
| Fleischextraktpaste | - | 8,0 | - | - |
| Lactose | 20,0 | 20,0 | 20,0 | 20,0 |
| Dikaliumhydrogenphosphat | 2,0 | 2,0 | 2,0 | 2,0 |
| Ammoniumcitrat | 2,0 | 2,0 | 2,0 | 2,0 |
| Mangan (II) sulfat | 0,05 | 0,05 | 0,05 | 0,05 |

Der pH-Wert wurde vor dem Autoklavieren (20 min bei 121°C) auf 6,2 eingestellt. Stickstoffquellen (Hefeextrakt, Pepton, Ammoniumcitrat) und Lactose wurden separat vom Rest autoklaviert, um Maillard-Reaktion zu vermeiden und anschließend unter der Sterilbank vereinigt, sodass sich die obigen Konzentrationen ergaben.

doppelte Vorkultur: 15 ml- Röhrchen mit 50 µl Glycerinkultur beimpft, 37°C, 50 rpm, angekippt liegend, über Nacht (16 h), davon 1 ml auf 15 ml frischen Bouillon (nochmal 10 h inkubiert)
Hauptkultur: 40 ml Medium mit 1,5 % der 2. Vorkultur inokuliert, 37°C, 50 rpm, 15 h
Zellernte: Zentrifugation bei 4°C, 25.000 g, 10 min, 1 x mit Puffer (50 mM Na-Phosphat pH 6,8) waschen

### B) Analytik

pH-Wert: im Zentrifugationsüberstand gemessen
Biotrockenmasse (BTM): Membranfiltermethode (Filter: Cellulose-Nitrat 0,45 µm, 15 ml Probe, mit 10 ml dest. W. waschen), Auswaage nach ca. 24h im Exsikkator
Zellaufschluss: anhand BTM-Ergebnis auf 0,015 g/ml mit Extraktionspuffer (50 mM Na-Phosphat pH 6,8, 20 % Glycerol,1 mM DTT) verdünnt, dann 3 x 10 min mit Zwischenkühlung auf Eis im Disruptor Genie (Aufschluss mit 0,2 g Glaskugeln (Ø 0,1 mm) plus 0,5 ml Zelllösung in einem 2 ml-Reaktionsgefäß), dann 10 min Zentrifugation (4°C, 10 min, 50.000 g) zur Abtrennung der Zellfragmente
Enzymassay: 22 mM o-NPG in 50 mM Na-Phosphat-Puffer pH 6,5 bei 35°C, 10 min, Abstoppen mit 0,4 M NaCO₃, Messung der Extinktion bei 420 nm (ε = 0,0049 µM⁻¹cm⁻¹) Eine Unit (U) wird definiert als die Enzymmenge, die unter den jeweiligen Reaktionsbedingungen 1 µmol o-Nitrophenol (o-NP) pro Minute freisetzt.

### C) Ergebnisse

Neben Caseinpepton wurde auch Sojapepton (je 10 g/L) als Alternative zu Fleischpepton untersucht, sowie ein basales Medium ohne komplexe N-Quelle. Die Ergebnisse sind in Fig. 4 dargestellt. Der Versuch erfolgte in Doppelbestimmung an unabhängigen Versuchstagen. Erwartungsgemäß korrelieren pH-Wert-Abfall und BTM, wobei das basale Medium das schlechteste Wachstum und damit auch die geringste Enzymausbeute (beta-Galactosidase) zeigt. Die beta-Galactosidase- Ausbeute pro Zelle ist bei Caseinpepton-Medium am höchsten (Fig. 4D). Bezogen auf das eingesetzte Volumen an Kulturmedium gibt es keinen Unterschied zum Standard-MRS- Medium (Fig. 4C). Ein Medium mit Sojapepton ist hinsichtlich Zell- und Enzymmasse nicht empfehlenswert.

### Beispiel 2: GOS-Synthese: Caseinpepton- vs. Fleisch-MRS-Extrakt

Die Enzymaktivität des Rohextraktes wurde bei der jeweiligen Temperatur mit o-NPG (22mM) in 50 mM Na-Phosphat-Puffer (Na₂HPO₄/NaH₂PO₄) pH 6,5 bestimmt (vgl. Bsp. 1). Daraus wurde die benötigte Menge an Enzymlösung (µl) zur Einstellung eines Enzym/Substrat (E/S)-Verhältnisses von 30 Units/Gramm Lactose bestimmt. Die daraus resultierende Verdünnung der Substratlösung wurde bei der Einwaage von Lactose (in 50 mM Na-Phosphat-Puffer pH 6,5) bzw. Süßmolkenpulver (in dest. Wasser) berücksichtigt, sodass sich immer eine finale Konzentration von 45 g/L Lactose ergab, was ca. 6,5 % Trockenmasse bei Molke entspricht. Die Lösungen wurden jeweils am Vortrag frisch angesetzt. Der pH-Wert der Molke wurde vor dem Versuch auf 6,5 eingestellt (original pH-Wert ca. 6,4). Die Reaktion wurde über einen Zeitraum von bis zu 31 h verfolgt. Zum Abstoppen der Reaktion wurden die entnommenen Proben hitzebehandelt (95°C, 5 min). Die GOS-Analytik erfolgte auf der Hi-Plex Na- Säule von Agilent Technologies mit RI-Detektion (Eluent 0,2 % Na-Azid in Wasser, Fluss 0,3 ml/min, Ofentemperatur 80°C).

Zunächst wurde geprüft, ob es quantitative Unterschiede in der GOS-Synthese zwischen den Extrakten aus dem Standard-MRS (Fleisch-MRS, Abk. F) und denen aus Caseinpepton-MRS (Abk. CP) gibt. Beide Extrakte verhielten sich ähnlich. Die Abweichung der maximalen GOS-Ausbeute liegt bei maximal 0,5 % (Tabelle 2).

**Tabelle 2: Maximale GOS-Ausbeute in % mit dem Extrakt aus Fleisch- bzw. Caseinpepton-MRS- Medium, Synthese bei 50°C**

| **Medium** | **CP-MRS (4 h)** | **F-MRS (3 h)** | **Δ** |
|---|---|---|---|
| Puffer | 15,9 | 15,4 | 0,5 |
| Molke | 14,8 | 14,5 | 0,3 |

Die GOS-Zusammensetzung wird jedoch beeinflusst (Tabelle 3). Die Verwendung des Extrakts aus Zellen, die in CP-MRS kultiviert wurden, führt zur vermehrten Bildung längerkettiger GOS (DP3 und DP4).

**Tabelle 3: GOS-Zusammensetzung (% der Gesamt-GOS) zum Zeitpunkt der maximalen GOS-Ausbeute mit dem Extrakt aus Fleisch- bzw. Caseinpepton-MRS- Medium, DP = Polymerisationsgrad (Kettenlänge), Synthese bei 50°C**

| **Medium** | **Extrakt aus...** | **DP4** | **DP3** | **DP2** |
|---|---|---|---|---|
| Puffer | F-MRS | 2,8 | 46,3 | 50,9 |
| Puffer | CP-MRS | 4,8 | 67,9 | 27,3 |
| Molke | F-MRS | 2,9 | 43,7 | 53,4 |
| Molke | CP-MRS | 5,2 | 62,8 | 32,0 |

### Beispiel 3: GOS-Synthese: Temperatureinfluss

Die GOS-Synthese wurde bei 50, 40, 30, 20 und 10°C mit einem Extrakt aus *Lactobacillus bulgaricus* Stamm LB4, welcher aus einer Kultivierung mit Caseinpepton-MRS erhalten wurde, durchgeführt.

In Fig. 5 sind die Ergebnisse in Puffer (50 mM Na-Phosphat-Puffer (Na₂HPO₄/NaH₂PO₄) pH 6,5) dargestellt. Die Ergebnisse in Süßmolke (pH eingestellt auf 6,5), dargestellt in Fig. 6, sind ähnlich, aber die Ausbeute ist etwas vermindert.

Die GOS-Ausbeute kann durch Temperatursenkung gesteigert werden. Das Maximum verschiebt sich jedoch zu längeren Reaktionszeiten, wie in Fig. 5/6A erkennbar ist. Fig. 5/6B zeigt, dass der Lactose-Abbau trotz des gleichen E/S-Verhältnisses von 30 U_{ONPG}/g_{Lactose} bei höherer Temperatur deutlich schneller ist. Die maximale GOS-Ausbeute wird unabhängig von der Temperatur bei ca. 70-80% Hydrolyse erreicht (Fig. 5/6C). Die Transgalactosylierungsaktivität (gemessen am Glucose/Galactose- Faktor, Fig. 5/6D) steigt mit abnehmender Temperatur.

In Abhängigkeit der Synthesetemperatur zeigt Fig. 7A die Zusammensetzung der Zucker in Molke zum Zeitpunkt der maximalen GOS-Ausbeute, Fig. 7B zeigt die GOS-Zusammensetzung zu diesem Zeitpunkt. Die Ergebnisse in Puffer sind ähnlich (nicht gezeigt).

Die GOS-Zusammensetzung wird von der Synthesetemperatur beeinflusst. Der Anteil an kurzkettigen GOS (Disaccharide) wird mit abnehmender Reaktionstemperatur höher. V.a. die erhöhte Synthese von GOS-Disacchariden (DP2) führt zur Steigerung der Ausbeute bei niedrigeren Temperaturen. Allerdings nimmt gleichzeitig auch der (absolute) Anteil an Trisacchariden (DP3) (und Tetrasacchariden (DP4)) ab, wie Fig. 7C zeigt.

### Beispiel 4: Inaktivierung des Enzyms

Eine einfach durchzuführende und kostengünstige Inaktivierungsmethode stellt die Hitzeinaktivierung dar. Dies erfordert allerdings ein dementsprechend geeignetes Temperaturprofil des Enzyms. Eine Inaktivierung des Enzyms im Produkt ist wichtig, da ansonsten im fertigen Produkt die Reaktionen weiterlaufen und die gebildeten GOS durch die hydrolytische Aktivität der beta-Galactosidasen wieder abgebaut werden würden. Bei Hitzeinaktivierung treten allerdings bei höheren Temperaturen Produktschädigungen auf. Hier laufen Maillard-Reaktionen ab, die durch Braunfärbung und Fehlgeschmack charakterisiert sind.

Um die Eignung einer Hitzeinaktivierung zur Enzymdenaturierung beurteilen zu können, wurde eine Inaktivierungskinetik in Puffer (50 mM Na-Phosphat pH 6,5) aufgenommen. Dazu wurde zuerst die Aktivität der Ausgangsprobe bestimmt (bei 35°C). Danach wurde die Enzym-Probe aufgeteilt und bei unterschiedlichen Temperaturen (von mind. 55°C bis max. 70°C) für bestimmte Zeit inkubiert (je nach Temperatur von maximal 5 bis 20 min). Danach wurden die Enzyme 5 min auf Eis gehalten und die verbleibende Aktivität (bei 35°C) des in der Probe vorhandenen Enzyms gemessen.

Mit der Formel t_{1/2}= In2/k_{d} kann die Halbwertszeit der beta-Galactosidase bestimmt werden, wobei k_{d} die Reaktionsgeschwindigkeitskonstante ist. Tabelle 4 gibt die ermittelten Halbwertszeiten für LB4 wider.

**Tabelle 4: Halbwertszeit (t_{1/2}) in min bei verschiedenen Temperaturen**

| **T in °C** | **t_{1/2} in min** |
|---|---|
| 70 | 0,87 |
| 65 | 4,10 |
| 60 | 10,45 |
| 55 | 64,38 |
| 70 | 0,96 |
| 65 | 4,67 |
| 60 | 18,09 |
| 55 | 55,90 |

Mit Hilfe des Arrhenius-Plots (In(k_{d}) vs. 1/T) wurden die Messwerte korreliert (R² = 0,987) und über die erhaltene Gleichung wurden bestimmte Temperatur-Zeit- Kombinationen ermittelt, welche in Fig. 8, zusammen mit den Linien gleichen Effekts für die Inaktivierung pathogener Keime, beta-Lactoglobulindenaturierung (als temperatursensitivstes Molkenprotein), Maillard-Reaktionen (als Parameter dient zum einen die Farbveränderung, da aus den Folgeprodukten der Maillard-Reaktion im letzten Schritt braune Verbindungen (Melanoidine) entstehen; zum anderen die HMF (Hydroxymethylfurfural)-Bildung), sowie Vitamin- bzw. Aminosäureschädigung (Daten aus "Lebensmittel- und Verfahrenstechnik, Molkereitechnologie" von H.G. Kessler, siehe auch Abbildungsbeschriftung) dargestellt sind.

Für die Keimabtötung muss man bei Temperatur/Zeit- Kombinationen arbeiten, die sich über den jeweiligen Geraden abspielen. Für die Inaktivierung der beta-Galactosidase gilt dies analog. Alle anderen Prozesse sind unerwünschte Reaktionen, sodass der Arbeitspunkt unterhalb der jeweiligen Linien liegen sollte. Wie Fig. 8 zeigt, kann eine beta-Lactoglobulindenaturierung von maximal 5 %, eine Lysin- bzw. Thiamin- Schädigung von max. 1 %, eine HMF-Bildung von max. 1 µmol/L eingehalten werden. Ebenso tritt keine Farbveränderung auf.

Zusammenfassend lässt sich festhalten, dass Hitzeinaktivierung (z.B. Kurzzeit, Hocherhitzung bzw. Ultrahocherhitzung) gut geeignet ist, um sowohl ein verkehrsfähiges Produkt zu erhalten (Keimabtötung), als auch die erfindungsgemäß sinnvolle Enzyminaktivierung zu realisieren.

## Patentansprüche

1. Verfahren zur Herstellung einer Galactooligosaccharid-haltigen Zusammensetzung, bei dem man eine von *L. bulgaricus* abgeleitete beta-Galactosidase bei einer Temperatur von 37°C oder weniger mit einer Lactose-haltigen Flüssigkeit ausgewählt aus der Gruppe umfassend Molke, Milch oder Puffer, bevorzugt Molke, inkubiert.

2. Verfahren zur Herstellung einer Galactooligosaccharid-haltigen Zusammensetzung mit einem definierten Anteil von DP4 Galactooligosacchariden und DP3 Galactooligosacchariden an allen Galactooligosacchariden, bei dem man eine von *L. bulgaricus* abgeleitete beta-Galactosidase mit einer Lactose-haltigen Flüssigkeit ausgewählt aus der Gruppe umfassend Molke, Milch oder Puffer, bevorzugt Molke, inkubiert, wobei man
a) die Lactose-haltige Flüssigkeit mit der beta-Galactosidase bei einer Temperatur von 10°C oder weniger inkubiert und der Anteil von DP4 Galactooligosacchariden 1 % oder weniger, bevorzugt 0,6 % oder weniger, und der Anteil von DP3 Galactooligosacchariden 22 % oder weniger, bevorzugt 20 % oder weniger beträgt;
b) die Lactose-haltige Flüssigkeit mit der beta-Galactosidase bei einer Temperatur von mehr als 10°C bis 20°C inkubiert und der Anteil von DP4 Galactooligosacchariden 0,5-2 %, bevorzugt 0,6-1,4 % beträgt und der Anteil von DP3 Galactooligosacchariden 18-37 %, bevorzugt 19,8 bis 34,5 % beträgt;
c) die Lactose-haltige Flüssigkeit mit der beta-Galactosidase bei einer Temperatur von mehr als 20°C bis 30°C inkubiert und der Anteil von DP4 Galactooligosacchariden 1-4 %, bevorzugt 1,4-3,4 % beträgt und der Anteil von DP3 Galactooligosacchariden 33-51 %, bevorzugt 34,5-48,9 % beträgt;
d) die Lactose-haltige Flüssigkeit mit der beta-Galactosidase bei einer Temperatur von mehr als 30°C bis 40°C inkubiert und der Anteil von DP4 Galactooligosacchariden 3-4,1 %, bevorzugt 3,4-3,7 % beträgt und der Anteil von DP3 Galactooligosacchariden 46-57 %, bevorzugt 48,9-55,2 % beträgt;
e) die Lactose-haltige Flüssigkeit mit der beta-Galactosidase bei einer Temperatur von mehr als 40° bis 50°C inkubiert und der Anteil von DP4 Galactooligosacchariden 3,5-5,5 %, bevorzugt 3,7-5,2 % beträgt und der Anteil von DP3 Galactooligosacchariden 53-65 %, bevorzugt 55,2-62,8 % beträgt; oder
f) die Lactose-haltige Flüssigkeit mit der beta-Galactosidase bei einer Temperatur von mehr als 50°C bis 60°C inkubiert und der Anteil von DP4 Galactooligosacchariden mehr als 5 %, bevorzugt mehr als 5,2 % beträgt und der Anteil von DP3 Galactooligosacchariden mehr als 60 %, bevorzugt mehr als 62,8 % beträgt.

3. Verwendung einer von *L. bulgaricus* abgeleiteten beta-Galactosidase zur Herstellung einer Galactooligosaccharid-haltigen Zusammensetzung in einer Lactose-haltigen Flüssigkeit ausgewählt aus der Gruppe umfassend Molke, Milch oder Puffer, bevorzugt Molke, bei einer Temperatur von 37°C oder weniger.

4. Verfahren nach Anspruch 1-2 oder die Verwendung nach Anspruch 3, wobei die Temperatur 30°C oder weniger beträgt,
zur Herstellung von Galactooligosacchariden oder einer Galactooligosaccharid-haltigen Zusammensetzung, welche bevorzugt einen Anteil von 4 % oder weniger, bevorzugt 3,4 % oder weniger DP4 Galactooligosacchariden und 51 % oder weniger, bevorzugt 48,9 % oder weniger DP3 Galactooligosacchariden an allen Galactooligosacchariden aufweist, wobei der Anteil der Galactooligosaccharide an dem Gesamtzucker mindestens 13 %, bevorzugt, mindestens 14 % beträgt.

5. Verfahren oder die Verwendung nach Anspruch 3, wobei die Temperatur 20°C oder weniger beträgt,
zur Herstellung von Galactooligosacchariden oder einer Galactooligosaccharid-haltigen Zusammensetzung, welche bevorzugt einen Anteil von 2 % oder weniger, bevorzugt 1,4 % oder weniger DP4 Galactooligosacchariden und 37 % oder weniger, bevorzugt 34,5 % oder weniger DP3 Galactooligosacchariden an allen Galactooligosacchariden aufweist, wobei der Anteil der Galactooligosaccharide an dem Gesamtzucker mindestens 15 %, bevorzugt, mindestens 16 % beträgt.

6. Verfahren oder die Verwendung nach Anspruch 4, wobei die Temperatur 10°C oder weniger beträgt,
zur Herstellung von Galactooligosacchariden oder einer Galactooligosaccharid-haltigen Zusammensetzung, welche bevorzugt einen Anteil von 1 % oder weniger, bevorzugt 0,6 % oder weniger DP4 Galactooligosacchariden und 22 % oder weniger, bevorzugt 20 % oder weniger DP3 Galactooligosacchariden an allen Galactooligosacchariden aufweist, wobei der Anteil der Galactooligosaccharide an dem Gesamtzucker mindestens 18 %, bevorzugt, mindestens 20 % beträgt.

7. Verfahren nach Anspruch 1 oder die Verwendung nach Anspruch 2, wobei die Temperatur mehr als 30 - 37°C beträgt,
zur Herstellung von Galactooligosacchariden oder einer Galactooligosaccharid-haltigen Zusammensetzung, welche bevorzugt einen Anteil von 3-4,1 %, bevorzugt 3,4-3,7 % DP4 Galactooligosacchariden und 46-57 %, bevorzugt 48,9-55,2 % DP3 Galactooligosacchariden an allen Galactooligosacchariden aufweist, wobei der Anteil der Galactooligosaccharide an dem Gesamtzucker mindestens 15 %, bevorzugt, mindestens 16 % beträgt.

8. Verfahren nach einem der Ansprüche 1, 2 oder 4-7 oder die Verwendung nach einem der Ansprüche 3-7, wobei die Molke ausgewählt ist aus der Gruppe umfassend Sauermolke, Süßmolke und Molkenpermeat, und einem Konzentrat aus Sauermolke, Süßmolke und Molkenpermeat, bevorzugt Süßmolke.

9. Verfahren nach einem der Ansprüche 1, 2 oder 4-8 oder die Verwendung nach einem der Ansprüche 3-8, wobei die beta-Galactosidase von Stamm LB4 abgeleitet ist.

10. Verfahren nach einem der Ansprüche 1, 2 oder 4-9 oder die Verwendung nach einem der Ansprüche 3-9, wobei die beta-Galactosidase mindestens 90 % Sequenzidentität zu beta-Galactosidase aus *L. bulgaricus* Stamm ATCC 11842 aufweist, bevorzugt 100 % Sequenzidentität.

11. Verfahren nach einem der Ansprüche 1, 2 oder 4-10 oder die Verwendung nach einem der Ansprüche 3-10, wobei man die Inkubation stoppt, wenn ein Hydrolysegrad der Lactose von 60-85 % erreicht ist, bevorzugt durch Hitzeinaktivierung.

12. Verfahren nach einem der Ansprüche 1, 2 oder 4-11 oder die Verwendung nach einem der Ansprüche 3-11, wobei die beta-Galactosidase aus in einem Caseinpeptide enthaltenden Medium, bevorzugt einem Caseinpepton-Medium kultivierten *L. bulgaricus* isoliert ist.

13. Verfahren, bevorzugt nach einem der Ansprüche 1, 2 oder 4-12, oder Verwendung, bevorzugt nach einem der Ansprüche 3-12, bei dem man
a) *L. bulgaricus* in einem Caseinpeptide enthaltenden Medium , bevorzugt, in einem Caseinpepton-Medium, kultiviert und beta-Galactosidase isoliert;
b) eine Lactose-haltige Flüssigkeit ausgewählt aus der Gruppe umfassend Molke, Milch oder Puffer, bevorzugt Molke, mit der isolierten beta-Galactosidase inkubiert;
c) die Inkubation stoppt, wenn ein Hydrolysegrad der Lactose von 60-85 % erreicht ist, bevorzugt durch Hitzeinaktivierung, und
d) optional Galactooligosaccharide aufreinigt und/oder Zutaten hinzufügt.

14. Verfahren nach einem der Ansprüche 1, 2 oder 4-13 oder die Verwendung nach einem der Ansprüche 3-13, wobei die Zusammensetzung ausgewählt ist aus der Gruppe umfassend Säuglingsnahrung, insbesondere Milchpulver, Kleinkindnahrung, Diätnahrung, Pharmazeutika, Nutrazeutika, Brei, Milchprodukte, Joghurt, Milchgetränke, Molkengetränke, Energy-Drinks, Fruchtsaft, Dessert, Snacks, Schokolade, Kekse, und Tierfutter.

15. Zusammensetzung, umfassend Molkenproteine und Galactooligosaccharide in einem Anteil der Galactooligosaccharide an dem Gesamtzucker von mindestens 13 % und, bevorzugt, isolierte beta-Galactosidase abgeleitet aus *L. bulgaricus,* welche bevorzugt mit einem Verfahren nach einem der Ansprüche 1, 2 oder 4-14 herstellbar ist,
wobei die Zusammensetzung ausgewählt ist aus der Gruppe umfassend
a) eine Zusammensatzung mit einen Anteil von 1 % oder weniger, bevorzugt 0,6 % oder weniger DP4 Galactooligosacchariden und 22 % oder weniger, bevorzugt 20 % oder weniger DP3 Galactooligosacchariden an allen Galactooligosacchariden, wobei der Anteil der Galactooligosaccharide an dem Gesamtzucker mindestens 18 %, bevorzugt, mindestens 20 % beträgt,
b) eine Zusammensatzung mit einen Anteil von 0,5-2 %, bevorzugt 0,6-1,4 % DP4 Galactooligosacchariden und 18-37 %, bevorzugt 19,8- 34,5 % DP3 Galactooligosacchariden an allen Galactooligosacchariden, wobei der Anteil der Galactooligosaccharide an dem Gesamtzucker mindestens 15 %, bevorzugt, mindestens 16 % beträgt,
c) eine Zusammensatzung mit einen Anteil von 1-4 %, bevorzugt 1,4-3,4 % DP4 Galactooligosacchariden und 33-51 %, bevorzugt 34,5-48,9 % DP3 Galactooligosacchariden an allen Galactooligosacchariden,
d) eine Zusammensatzung mit einen Anteil von 3-4,1 %, bevorzugt 3,4-3,7 % DP4 Galactooligosacchariden und 46-57 %, bevorzugt 48,9-55,2 % DP3 Galactooligosacchariden an allen Galactooligosacchariden, und
e) eine Zusammensatzung mit einen Anteil von 3,5-5,5 %, bevorzugt 3,7-5,2 % DP4 Galactooligosacchariden und 53-65 %, bevorzugt 55,2-62,8 % DP3 Galactooligosacchariden an allen Galactooligosacchariden, und
f) eine Zusammensatzung mit einen Anteil von mehr als 5 %, bevorzugt mehr als 5,2 % DP4 Galactooligosacchariden und mehr als 60 %, bevorzugt mehr als 62,8 % DP3 Galactooligosacchariden an allen Galactooligosacchariden.
